# EUROPÄISCHE PATENTANMELDUNG

(11) **EP 3 132 805 A1**
(43) Veröffentlichungstag der Anmeldung: **22.02.2017**
(21) Anmeldenummer: 15168482.6
(22) Anmeldetag: 20.05.2015
(51) Int. Cl.: A61L 2/26, A61L 2/07, A61B 50/30

(54) **STERILISATIONSCONTAINER MIT FILTERBLATTHALTERUNG**

(71) Anmelder: Interlock Medizintechnik GmbH, 23738 Lensahn (DE)
(72) Erfinder: Steinwärder, Dirk, 22301 Hamburg (DE)
(74) Vertreter: Meyer, Ludgerus

(57) **Zusammenfassung**

Die Erfindung betrifft einen Sterilisationscontainer, insbesondere zur Aufnahme oder Lagerung von Sterilisationsgut, mit einem wannenförmigen Unterteil zur Aufnahme des Sterilisationsgutes und einer auf das Unterteil aufsetzbaren Deckelplatte (1), welche mit einer Halterung zur Befestigung eines Filtermaterials zur Abdeckung von Strömungskanälen (5) in der Deckelplatte versehen ist, wobei die Strömungskanäle (5) auf der dem Filtermaterial gegenüberliegenden Seite der Deckelplatte (1) mit Abstand von dem Bereich der Strömungskanäle mit einer Schutzplatte (2, 13) abdeckbar sind. Erfindungsgemäß ist die Schutzplatte (2, 13) über wenigstens eine lösbare Halterung unter Klemmkraft oder magnetischer Kraft an der Deckelplatte (1) befestigbar, wobei die Schutzplatte (2, 13) über Stützelemente (12, 26) gegenüber der Deckelplatte (1) auf einen solchen freien Abstand gehalten wird, dass eine Gas- oder Dampfströmung zwischen Deckelplatte (1) und Schutzplatte (2), durch die Strömungskanäle (5) und durch das Filtermaterial gewährleistet ist. (Fig. 1)

## Beschreibung

Die Erfindung betrifft einen Sterilisationscontainer, insbesondere zur Aufnahme oder Lagerung von Sterilisationsgut, wie z. B. zu sterilisierende medizinische Geräte und Instrumente, nach dem Oberbegriff des Anspruchs 1.

In medizinischen Einrichtungen und Krankenhäusern wird eine Vielzahl von Instrumenten und Geräten verwendet, die regelmäßig zu sterilisieren sind. Kleinere Geräte und Instrumente werden dabei in der Regel in einen Behälter eingelegt, der aus einem wannenförmigen Unterteil zur Aufnahme des Sterilisationsgutes und eine auf das Unterteil aufsetzbare gasdurchlässige Deckelplatte gebildet ist. Die Deckelplatte weist dazu Strömungskanäle auf, durch die Sterilisiermittel (Agens) hindurchgeführt werden kann. An der Unterseite der Deckelplatte ist ein austauschbares Filtermaterial befestigt. Zur Abdeckung der Strömungskanäle gegen das Eindringen von Fremdkörpern in das wannenförmige Unterteil und um es zu ermöglichen, mehrere Sterilisationscontainer zu stapeln, ist die Deckelplatte häufig mit Abstand von dem Bereich der Strömungskanäle mit einer zusätzlichen Schutzplatte abdeckbar. Die Schutzplatte ist an der Deckelplatte befestigt.

Aus der DE 297 20 450 U1 ist ein Sterilisationscontainer bekannt, bei dem eine mit einer speziellen Struktur versehene Abdeckplatte an der Deckelplatte befestigt wird, wobei die Struktur in eine äquivalente Struktur der Deckelplatte angreift, so dass sich Strömungskanäle bilden, die mäanderförmig verlaufen. Die Herstellung eines solchen Sterilisationscontainers ist entsprechend aufwendig. Die Befestigung des Deckels und der Abdeckplatte erfolgt über eine zentrale Verschraubung.

Der Erfindung liegt die Aufgabe zugrunde, einen Sterilisationscontainer der angegebenen Art, insbesondere zur Aufnahme oder Lagerung von Sterilisationsgut, derart zu verbessern, dass dessen Herstellung vereinfacht ist, eine einfache Trennung oder Verbindung von Deckelplatte und Schutzplatte möglich ist, aber gleichwohl eine Stapelbarkeit gewährleistet bleibt.

Diese Aufgabe wird durch die im Anspruch 1 angegebene Erfindung gelöst. Vorteilhafte Weiterbildungen der Erfindung sind in Unteransprüchen angegeben.

Die Erfindung geht aus von einem Sterilisationscontainer mit einem wannenförmigen Unterteil zur Aufnahme des Sterilisationsgutes und einer auf das Unterteil aufsetzbaren Deckelplatte, welche mit einer unterseitigen Halterung zur Befestigung eines Filtermaterials zur Abdeckung von Strömungskanälen versehen ist, wobei die Strömungskanäle auf der dem Filtermaterial gegenüberliegenden Seite der Deckelplatte mit Abstand von dem Bereich der Strömungskanäle mit einer Schutzplatte abdeckbar sind.

Gemäß der Erfindung ist die Schutzplatte über wenigstens eine lösbare Halterung an der Deckelplatte befestigbar, wobei die Schutzplatte über Stützelemente gegenüber der Deckelplatte auf einen solchen freien Abstand gehalten wird, dass eine Gas- oder Dampfströmung zwischen Deckelplatte und Schutzplatte, durch die Strömungskanäle und durch das Filtermaterial ermöglicht ist.

Beim Gegenstand der Erfindung können die Strömungskanäle einfach ausgebildet sein, z. B. als eine Vielzahl von einfachen Bohrungen in der Deckelplatte. Die Bohrungen sind durch die Schutzplatte abgedeckt, wobei ein freier Abstand zwischen Deckelplatte und Schutzplatte eingehalten wird, um die Gas- oder Dampfströmung zwischen Deckelplatte und Schutzplatte nicht zu behindern. Die Gas- oder Dampfströmung tritt durch die Strömungskanäle in das Innere des Sterilisationscontainers, wobei sie ein Filtermaterial an der Unterseite der Deckelplatte passiert. Das Filtermaterial wird durch einen Filterhalter an der Unterseite der Deckelplatte befestigt.

Die Schutzplatte ist vorzugsweise als kreis- oder rechteckförmige Platte ausgebildet, welche die verteilt in der Deckelplatte angeordneten Strömungskanäle mit freiem Abstand zur Deckelplatte abdeckt. Vorzugsweise ist die Schutzplatte als ebene Platte ausgebildet, welche randseitige Stützelemente enthält, die bei Befestigung der Schutzplatte an der Deckelplatte den freien Abstand bilden, indem die Stützelemente die Deckelplatte kontaktieren. Die Stützelemente sind insbesondere als randseitige Abbiegungen der Schutzplatte ausgebildet, die bei Befestigung der Schutzplatte an der Deckelplatte an die Deckelplatte anstoßen und damit punktuelle Kontaktpunkte bilden, die die freie Strömung zwischen Deckelplatte und Schutzplatte aber nicht behindern. In einer alternativen Ausführungsform weist die Deckelplatte im Bereich der Schutzplatte mehrere Erhebungen auf, gegen die eine ebene Schutzplatte anstoßen kann. Auch in diesem Fall bleibt ein freier Abstand zwischen Deckelplatte und Schutzplatte gewährleistet.

In bevorzugter Ausgestaltung der Erfindung ist die Schutzplatte entweder über eine Klemmhalterung an der Deckelplatte befestigbar oder sie kann über eine magnetische Halterung an der Deckelplatte befestigt werden. In der Ausbildungsform einer Klemmhalterung ist die Schutzplatte über einen zentralen lösbaren Haltestift an der Deckelplatte befestigbar, wobei an der Schutzplatte über eine zentrale Bohrung ein erstes Ende des Haltestifts befestigt ist. Das zweite Ende des Haltestifts wird durch eine zentrale Bohrung der Deckelplatte geführt, wobei der Haltestift einen flexiblen Klemmring aufweist, der beim Aufsetzen der Schutzplatte auf die Deckelplatte die Unterseite der Deckelplatte hintergreift und damit die Schutzplatte an der Deckelplatte arretiert. Der Haltestift weist ferner an dem der Schutzplatte abgewandten Ende einen Ansatz auf, welcher eine an sich bekannte Halterung für das Filtermaterial bildet. Es ist daher nicht erforderlich, an der Unterseite der Deckelplatte eine gesonderte Halterung für das Filtermaterial anzubringen. Der Haltering ist vorzugsweise als flexibler O-Ring gebildet, der in einer umlaufenden Nut des Haltestifts aufgenommen ist.

Die Befestigung des Filtermaterials an dem Haltestift hat auch den Vorteil, dass der Haltestift und damit die Schutzplatte nicht von der Deckelplatte entfernt werden können, solange das Filtermaterial bzw. desren Halterung an dem Haltestift befestigt ist. Erst nach Lösen der Halterung kann die Schutzplatte über den Haltestift von der Deckelplatte entfernt werden, indem der durch den Klemmring verursachte Widerstand überwunden wird.

In einer zweiten Ausführungsform wird die Schutzplatte über eine Magnetverbindung an der Deckelplatte befestigt. Hierzu enthält die Deckelplatte einen zentralen ring- oder scheibenförmigen Magneten mit einem zentralen Schraubansatz, der durch eine zentrale Bohrung der Deckelplatte hindurchgeführt werden kann. Auf der Rückseite der Deckelplatte lässt sich der Magnet mit einem Haltestift verbinden, so dass der Magnet fest an der Deckelplatte angeordnet werden kann. Der Haltestift kann ebenfalls, wie bei der ersten Ausführungsform, mit einem Ansatz versehen sein, über den das Filtermaterial bzw. deren Halterung an der Unterseite der Deckelplatte befestigt werden kann.

Die Schutzplatte weist vorzugsweise eine zentrale Bohrung auf, in die der Magnet eintreten kann. Die Oberseite der Schutzplatte enthält eine flanschförmige Ausnehmung zur Aufnahme einer magnetisierbaren Halteplatte. Diese kann über mehrere Halteschrauben oder mittels anderer entsprechender Befestigungsmittel, z. B. Nieten, an der Schutzplatte befestigt sein. Die Halteplatte gewährleistet eine ausreichende magnetische Haltekraft zur Befestigung der Schutzplatte, insbesondere wenn die Schutzplatte, wie es üblich ist, aus Aluminium oder einem anderen nicht magnetisierbarem Material besteht.

Während die axiale Dicke des verwendeten Magneten bereits einen definierten Abstand zwischen Deckelplatte und Schutzplatte bestimmt, kann auch in dieser Ausführungsform die Schutzplatte randseitige Stützelemente enthalten oder die Deckelplatte entsprechende Erhebungen. Dadurch wird verhindert, dass die Schutzplatte an einer Seite gegen die Deckelplatte gedrückt werden könnte und damit die gegenüberliegende Seite entsprechend abgehoben wird, so dass die Haltefunktion nicht mehr gewährleistet sein würde.

Der Sterilisationscontainer kann als runder oder rechteckförmiger Behälter ausgebildet sein, wobei eine zentrale Schutzplatte vorgesehen ist. Bei einem größeren Container können auch mehrere gleichartige Bereiche mit Strömungskanälen ausgebildet sein, auf die untereinander gleichartigen Schutzplatten aufgesetzt werden können.

Die Erfindung wird nachstehend anhand eines Ausführungsbeispiels näher erläutert. Es zeigen:
- Fig. 1: eine perspektivische Explosionsansicht einer ersten Ausführungsform der Erfindung,
- Fig. 2a - c: eine Deckelplatte in drei Ansichten zur Verwendung mit der ersten oder einer zweiten Ausführungsform der Erfindung,
- Fig. 3a - c: eine Schutzplatte gemäß der ersten Ausführungsform in drei Ansichten,
- Fig. 4a - c: einen Haltestift gemäß der ersten Ausführungsform der Erfindung in drei Ansichten,
- Fig. 5: einen Klemmring,
- Fig. 6: eine Schnittansicht der ersten Ausführungsform im zusammengesetzten Zustand,
- Fig. 7: eine perspektivische Explosionsansicht der zweiten Ausführungsform der Erfindung,
- Fig. 8a - c: eine Schutzplatte der zweiten Ausführungsform in drei Ansichten,
- Fig. 9a, b: einen Magneten in zwei Ansichten,
- Fig. 10a - c: einen Stift in drei Ansichten,
- Fig. 11: eine Halteplatte, und
- Fig. 12: eine Schnittansicht durch die zweite Ausführungsform im zusammengesetzten Zustand.

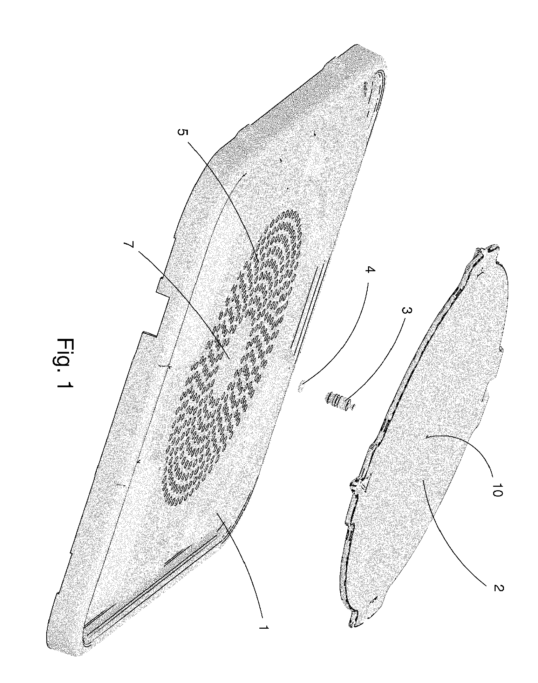

Die in Fig. 1 dargestellte Explosionsansicht eines ersten Ausführungsbeispiels der Erfindung zeigt eine im Wesentlichen rechteckförmige Deckelplatte 1 zur Befestigung an einem nicht dargestellten wannenförmigen Unterteil des Sterilisationscontainers. Auf die Deckelplatte 1 ist eine Schutzplatte 2 mittels eines Haltestifts 3 aufsetzbar. Der Haltestift 3 wird an der Schutzplatte 2 befestigt. Der Haltestift 3 lässt sich zusammen mit einem in eine Nut des Haltestifts 3 eingelegten Klemmring 4 durch eine zentrale Bohrung 7 der Deckelplatte 1 hindurchführen und damit die Schutzplatte 2 an der Deckelplatte 1 befestigen. Zur Vermeidung von Beschädigungen der zentralen Bohrung nach häufiger Einführung und Entnahme des Haltestifts kann die Bohrung eine Verstärkung enthalten, vorzugsweise eine Buchse aus stärkerem Metall als das Metall der Deckelplatte, wobei die Buchse in der Deckelplatte auch schwenkbar gelagert sein kann.

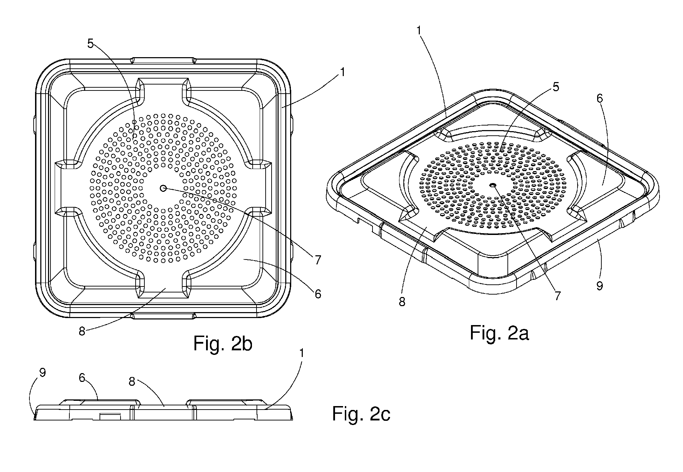

Die Figuren 2a - 2c zeigen die Deckelplatte 1 in verschiedenen Ansichten. In Fig. 2a ist die Deckelplatte in perspektivischer Ansicht dargestellt. Die Deckelplatte 1 enthält einen umlaufenden Deckelrand 9, der auf den Rand des nicht dargestellten wannenförmigen Unterteils des Sterilisationscontainers aufsetzbar ist. Die Deckelplatte 1 weist an den vier Ecken Erhebungen 6 auf, die von deDeckeloberseite 8 vorstehen. Dieser weist eine Vielzahl von Bohrungen auf, die als Strömungskanäle 5 ausgebildet sind. Im Zentrum der Deckelplatte 1 befindet sich eine Bohrung 7, über die die Schutzplatte 2 befestigt werden kann. Die Strömungskanäle 5 sind ringförmig angeordnet und nehmen nahezu den gesamten inneren Bereich der Deckelplatte ein, so dass diese als Siebplatte angesehen werden kann. Fig. 2c zeigt die Anordnung der Erhebungen 6 in den jeweiligen Ecken der Deckelplatte 1 in Seitenansicht. Zwischen den Erhebungen liegt die ebene Deckeloberseite 8, die zwischen den Erhebungen 6 bis zum Rand der Deckelplatte reicht.

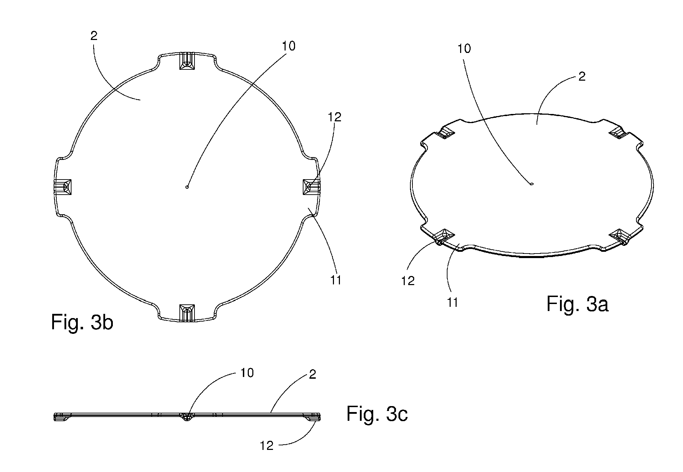

Die Figuren 3a - 3c zeigen die Schutzplatte 2 zum Aufsetzen auf die Deckelplatte 1. Die Schutzplatte 2 enthält eine zentrale Bohrung 10 zur Befestigung des Haltestifts 3. Am Rand der Schutzplatte befinden sich vier Ansätze 11, die beim Aufsetzen der Schutzplatte 2 auf die Deckelplatte 1 jeweils zwischen zwei Erhebungen 6 eingreifen können. Ferner befinden sich an den jeweiligen Ansätzen 11 Stützelemente 12, die von der Unterseite der Schutzplatte vorstehen und als Abbiegungen der Schutzplatte 2 ausgebildet sind. Fig. 3c zeigt, in welcher Weise die Stützelemente 12 von der Unterseite der Schutzplatte 2 abstehen. Wenn eine derartige Schutzplatte 2 auf eine Deckelplatte 1 gemäß Fig. 2 aufgesetzt wird, kontaktieren die Stützelemente 12 die Deckeloberseite 8 der Deckelplatte 1 im Bereich zwischen zwei Erhebungen 6. Die Höhen der Erhebungen 6 und der Stützelemente 12 sind so bemessen, dass die Oberflächen der Erhebungen 6 sowie der Schutzplatte 2 eine durchgehende Ebene bilden. Da die Stützelemente 12 einen Abstand der Schutzplatte 2 gegenüber der Deckelplatte 1 bewirken, befindet sich zwischen Deckelplatte 1 und Schutzplatte 2 ein definierter Raum, durch den ein Gas- oder Dampfstrom geführt werden kann, der über die Strömungskanäle 5 in das Innere des Sterilisationsbehälters eintreten kann.

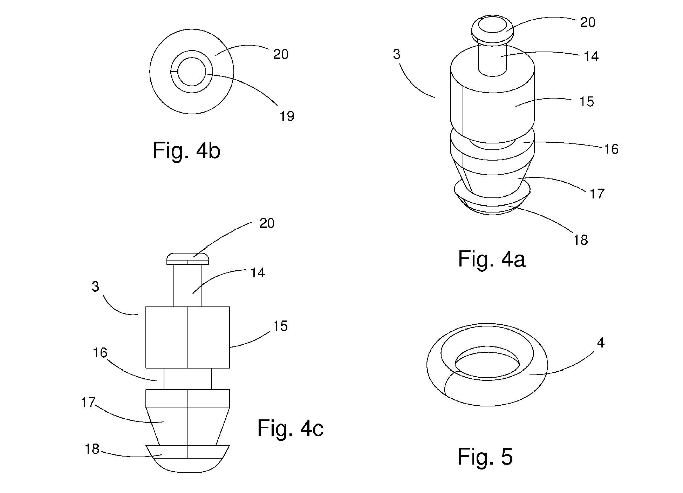

Die Fig. 4a - 4c zeigen den Haltestift 3 in vergrößerter Darstellung und unterschiedlichen Ansichten. Der Haltestift 3 enthält einen oberseitigen Hals 14, der durch die zentrale Bohrung 10 der Schutzplatte 2 hindurchgeführt werden kann. Der Hals 14 weist ein Innengewinde 19 auf, in das eine Schraube 20 eingeschraubt werden kann, um damit den Stift 3 an der Schutzplatte 2 zu befestigen. Der Körperbereich 15 definiert im Wesentlichen den Abstand zwischen Schutzplatte 2 und Deckelplatte 1, wobei jedoch das untere Ende des Körperbereichs 15 durch die zentrale Bohrung 7 der Deckelplatte geführt ist.

Unterhalb des Körperbereichs 15 befindet sich eine ringförmig umlaufende Nut 16, in die ein Klemmring 4, insbesondere eine O-Ring, einsetzbar ist. Der Klemmring 16 ragt von der Oberfläche des Körperbereichs 15 vor und hintergreift beim Einsetzen des Haltestiftes 3 in die Deckelplatte 1 die Unterseite der Deckelplatte rings um die zentrale Bohrung 7. Dadurch lässt sich der Haltestift 3 in der zentralen Bohrung 7 der Deckelplatte 1 verankern.

Am unteren Ende des Haltestiftes 3 befindet sich noch ein Konus 17 mit einem Ansatz 18, auf den in bekannter Weise eine nicht dargestellte Halterung zur Aufnahme eines Filtermaterials aufsetzbar ist. Die Halterung verhindert dabei, dass der Haltestift 3 aus der Bohrung 7 der Deckelplatte herausgehoben werden kann. Erst wenn die Halterung für den Filter entfernt ist, kann der Haltestift 3 nach Überwindung der Klemmkraft des Klemmrings aus der Deckelplatte entfernt werden. Der Klemmring 4 ist in Fig. 5 dargestellt.

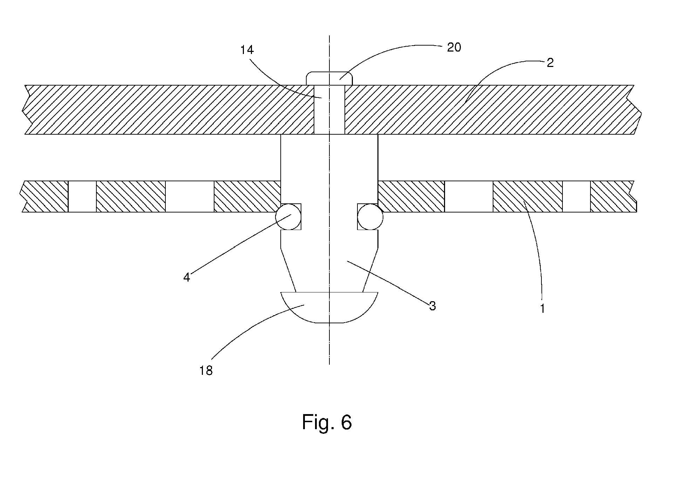

Fig. 6 zeigt eine Schnittansicht durch eine komplettierte Deckelplatte. Der Haltestift 3 ist durch die Deckelplatte 1 geführt und hintergreift mit seinem Klemmring 4 den Rand der Bohrung der Deckelplatte 1. Der Hals 14 ist durch die Schutzplatte 2 hindurchgeführt. Mittels der Schraube 20 ist der Haltestift 3 an der Schutzplatte 2 befestigt.

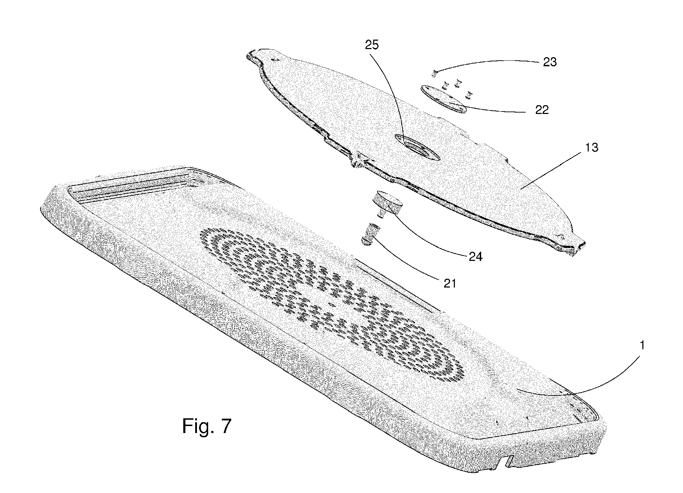

Fig. 7 zeigt eine Explosionsansicht der zweiten Ausführungsform der Erfindung, bei der die Schutzplatte 13 über eine magnetische Halterung an der Deckelplatte 1 befestigbar ist. Die magnetische Halterung besteht aus einem Magneten 24, der auf der Unterseite der Deckelplatte 1 mit einem Haltestift 21 verschraubbar ist. Dieser dient in gleicher Weise wie bei der ersten Ausführungsform auch zur Befestigung der Halterung für das Filtermaterial.

Der Magnet 24 tritt in eine zentrale Bohrung der Schutzplatte 13 ein. An der Oberseite der Schutzplatte 13 ist eine flanschförmige Ausnehmung 25 ausgebildet, in der eine magnetisierbare Halteplatte 22 mittels Schrauben 23, Nieten oder auch durch eine temperatur- und gasfeste Verklebung befestigt ist.

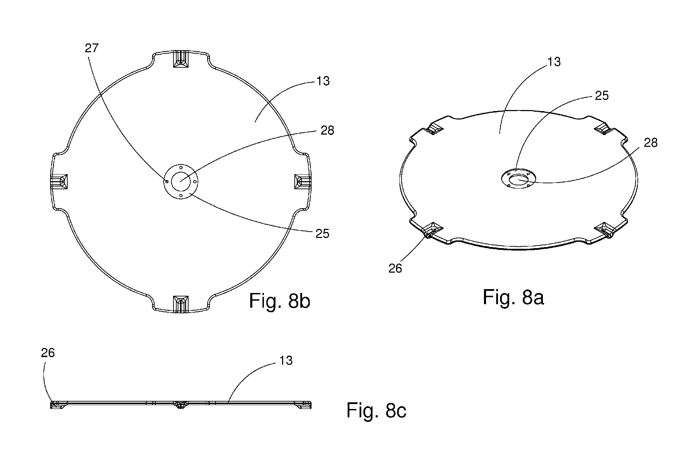

Die Figuren 8a - 8c zeigen eine Schutzplatte 13 der zweiten Ausführungsform. Die Schutzplatte 13 enthält ebenfalls wie in der ersten Ausführungsform randseitige Stützelemente 26, die einen definierten Abstand zur Oberseite der Deckelplatte 1 ermöglichen. Die bei der zweiten Ausführungsform verwendete Deckelplatte entspricht der in den Figuren 2a - c dargestellten Deckelplatte der ersten Ausführungsform.

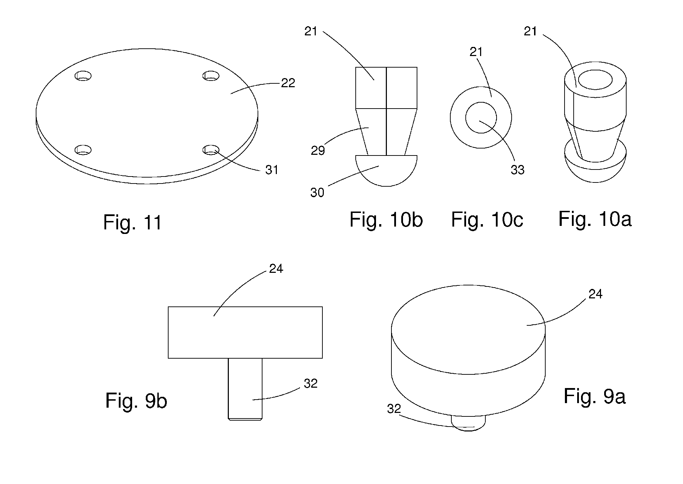

Die in den Figuren 8a und 8b dargestellte Schutzplatte 13 enthält eine zentrale Bohrung 28 zur Aufnahme des Magneten 24 sowie eine die Bohrung 28 umgebende Ausnehmung 25, in die eine gemäß Fig. 11 ausgebildete Halteplatte 22 einsetzbar ist. Die Halteplatte 22 besteht aus magnetisierbarem Material, insbesondere Edelstahl, um die nötige Haltekraft gegenüber dem Magneten 24 erreichen zu können, da das Material des Sterilisierungsbehälters selbst in der Regel nicht magnetisierbar ausgebildet ist.

Die Figuren 9a und 9b zeigen zwei Ansichten des Magneten 24 mit Schraubansatz 32 zur Befestigung an der Deckelplatte. Der Magnet ist scheibenförmig ausgebildet und besteht vorzugsweise aus seltenen Erden, um eine ausreichende magnetische Anziehungskraft zu erreichen und die Temperaturbeständigkeit zu garantieren.

Die Figuren 10a - 10c zeigen verschiedene Ansichten des Haltestifts 21. Dieser weist eine Gewindebohrung 33 auf, um ihn mit dem Schraubansatz 32 des Magneten 24 verbinden zu können. Die Oberseite des Haltestifts 21 kommt beim Verschrauben mit dem Schraubansatz 32 in Kontakt mit der Unterseite der Deckelplatte 1.

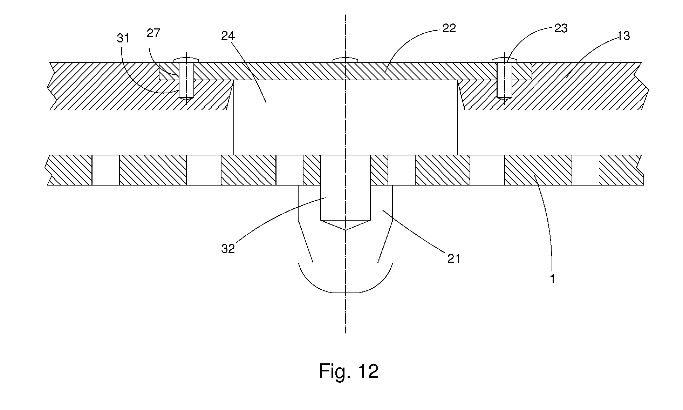

Fig. 12 zeigt eine Schnittansicht durch eine zusammengesetzte zweite Ausführungsform. Es ist deutlich zu erkennen, dass der Haltestift 21 gegen die Unterseite der Deckelplatte 1 stößt, wobei der Magnet 24 an dem Haltestift 21 über einen Schraubansatz 32 verschraubt ist. Der Magnet 24 ist in eine Bohrung der Schutzplatte 13 eingeführt, wobei die Oberseite des Magneten von der Oberfläche der Schutzplatte 13 zurückspringt. Auf die Oberseite der Schutzplatte 13 lässt sich die Halteplatte 22 in eine entsprechend vorbereitete Ausnehmung 25 der Schutzplatte 13 einsetzen, wobei die Halteplatte 22 über mehrere Schrauben 23 und Schraublöcher 27 oder Nieten in den Bohrungen 31 an der Schutzplatte 13 befestigt ist.

**Bezugszeichen**

| | | | |
|---|---|---|---|
| 1 | Deckelplatte | 18 | Ansatz |
| 2 | Schutzplatte | 19 | Gewinde |
| 3 | Haltestift | 20 | Schraube |
| 4 | Klemmring | 21 | Haltestift |
| 5 | Strömungskanäle | 22 | Halteplatte |
| 6 | Erhebungen | 23 | Schrauben |
| 7 | Bohrung in Deckelplatte | 24 | Magnet |
| 8 | Deckeloberseite | 25 | Ausnehmung |
| 9 | Deckelrand | 26 | Stützelemente |
| 10 | Bohrung in Schutzplatte | 27 | Schraublöcher |
| 11 | Ansatz | 28 | Bohrung |
| 12 | Stützelement | 29 | Konus |
| 13 | Schutzplatte | 30 | Ansatz |
| 14 | Hals | 31 | Bohrungen |
| 15 | Körperbereich | 32 | Schraubansatz |
| 16 | Nut | 33 | Gewindebohrung |
| 17 | Konus | | |

## Patentansprüche

1. Sterilisationscontainer, insbesondere zur Aufnahme oder Lagerung von Sterilisationsgut, mit einem wannenförmigen Unterteil zur Aufnahme des Sterilisationsgutes und einer auf das Unterteil aufsetzbaren Deckelplatte (1), welche mit einer Halterung zur Befestigung eines Filtermaterials zur Abdeckung von Strömungskanälen (5) in der Deckelplatte versehen ist, wobei die Strömungskanäle (5) auf der dem Filtermaterial gegenüberliegenden Seite der Deckelplatte (1) mit Abstand von dem Bereich der Strömungskanäle mit einer Schutzplatte (2) abdeckbar sind, **dadurch gekennzeichnet, dass** die Schutzplatte (2, 13) über wenigstens eine lösbare Halterung an der Deckelplatte (1) befestigbar ist, wobei die Schutzplatte (2, 13) über Stützelemente (12, 26) gegenüber der Deckelplatte (1) auf einen solchen freien Abstand gehalten wird, dass eine Gas- oder Dampfströmung zwischen Deckelplatte (1) und Schutzplatte (2, 13), durch die Strömungskanäle (5) und durch das Filtermaterial ermöglicht ist.

2. Sterilisationscontainer nach Anspruch 1, **dadurch gekennzeichnet, dass** die Schutzplatte (2, 13) als im Wesentlichen kreis- oder rechteckförmige Platte ausgebildet ist, welche die verteilt in der Deckelplatte angeordneten Strömungskanäle (5) mit freiem Abstand zur Deckelplatte (1) abdeckt.

3. Sterilisationscontainer nach Anspruch 1 oder 2, **dadurch gekennzeichnet, dass** die Schutzplatte (2, 13) als ebene Platte ausgebildet ist, welche randseitige Stützelemente (12, 26) enthält, die bei Befestigung der Schutzplatte (2) an der Deckelplatte (1) diese zur Einhaltung des freien Abstandes kontaktieren.

4. Sterilisationscontainer nach Anspruch 1 oder 2, **dadurch gekennzeichnet, dass** die Schutzplatte (2, 13) als ebene Platte ausgebildet ist und dass die Deckelplatte Erhebungen aufweist, die bei Befestigung der Schutzplatte (2) an der Deckelplatte (1) einen freien Abstand zwischen dem zentralen Bereich der Schutzplatte (2, 13) und der Deckelplatte (1) gewährleisten.

5. Sterilisationscontainer nach Anspruch 1 oder 2, **dadurch gekennzeichnet, dass** die Schutzplatte (2) über eine Klemmhalterung an der Deckelplatte (1) befestigbar ist.

6. Sterilisationscontainer nach Anspruch 5, **dadurch gekennzeichnet, dass** die Schutzplatte (2) über einen zentralen lösbaren Haltestift (3) an der Deckelplatte (1) befestigbar ist, wobei ein erstes Ende des Haltestifts (3) an der Schutzplatte (2) über eine zentrale Bohrung (10) befestigt ist, dass der Haltestift (3) einen flexiblen Klemmring (4) aufweist, dass ein zweites Ende des Haltestifts (3) durch eine zentrale Bohrung (10) der Deckelplatte (1) hindurchführbar ist, dass die Schutzplatte (2) beim Aufsetzen auf die Deckelplatte (1) durch den flexiblen Klemmring (4) an der Unterseite der Deckelplatte (1) arretiert wird, und dass der Haltestift (3) am zweiten Ende einen Ansatz (18) aufweist, welcher die Halterung für das Filtermaterial bildet.

7. Sterilisationscontainer nach Anspruch 1 oder 2, **dadurch gekennzeichnet, dass** die Schutzplatte (2) über eine magnetische Halterung an der Deckelplatte (1) befestigbar ist.

8. Sterilisationscontainer nach Anspruch 7, **dadurch gekennzeichnet, dass** die Deckelplatte (1) über einen zentralen gegen die Schutzplatte (13) gerichteten Magneten (24) mit der Schutzplatte (13) verbindbar ist, wobei der Magnet (24) mit einem zentralen Ansatz (32) versehen ist, der durch eine Bohrung (28) der Deckelplatte (1) hindurchführbar ist und an dem an der Unterseite der Deckelplatte (1) ein Haltestift (21) befestigbar ist, der einen Ansatz (30) aufweist, welcher die Halterung für das Filtermaterial bildet.

9. Sterilisationscontainer nach Anspruch 8, **dadurch gekennzeichnet, dass** der Magnet (24) in eine zentrale Bohrung (28) der Schutzplatte (13) einführbar ist, wobei die Bohrung (28) an der Oberseite der Schutzplatte (13) mit einer magnetisierbaren Halteplatte (22) versehen ist.

10. Sterilisationscontainer nach Anspruch 7, **dadurch gekennzeichnet, dass** die Deckelplatte (1) und die Schutzplatte (13) aus nicht magnetisierbarem Material gebildet sind.

11. Sterilisationscontainer nach Anspruch 1, **dadurch gekennzeichnet, dass** die Deckelplatte (1) zwei gleichartig ausgebildete Bereiche mit Strömungskanälen enthält, auf die Schutzplatten (2, 13) nach einem oder mehreren der vorhergehenden Ansprüche aufbringbar sind.
